(19) Europäisches Patentamt
European Patent Office
Office européen des brevets

(11) EP 4 509 464 A1

(12) EUROPEAN PATENT APPLICATION

(43) Date of publication:
**19.02.2025 Bulletin 2025/08**

(21) Application number: **23020379.6**

(22) Date of filing: **14.08.2023**

(51) International Patent Classification (IPC):
***C01B 33/18*** *(2006.01)*

(52) Cooperative Patent Classification (CPC):
**C01B 33/18;** C01P 2004/60; C01P 2006/10; C01P 2006/12

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Lygin, Alexander**
**64347 Griesheim (DE)**

(72) Inventor: **Lygin, Alexander**
**64347 Griesheim (DE)**

# (54) PRECIPITATED SILICA FOR IMPROVED ADSORPTION OF VOLATILE ORGANIC COMPOUNDS

(57) Dry mixture comprising from 10 % to 99 % by weight of a precipitated silica with a BET surface area of from 30 $m^2$/g to 700 $m^2$/g and having a silanol group density $d_{SiOH}$ determined by reaction of the pre-dried silica with lithium aluminum hydride of from 1.55 to 3.65 SiOH/$nm^2$and 1 wt% to 90 wt% of at least one organic compound, comprising at least one oxygen (O) and/or nitrogen (N) atom and having a vapour pressure of at least 0.01 kPa at 293.15 K, adsorbed on the precipitated silica, the process for manufacturing thereof and the use thereof as food and/or animal feed additive.

EP 4 509 464 A1

## Description

### Field of the invention

Present invention relates to a dry mixture comprising precipitated silica and a volatile organic compound adsorbed thereon, a process for preparation such a mixture and the use thereof, especially as a food or animal feed additive.

### Description of the prior art and the technical problem

Various organic compounds are often adsorbed on carrier materials based on precipitated silica, the resulting adsorbate/carrier systems being used e.g. as food or animal feed additives.

Thus, EP1241136A2 discloses preparation of precipitated silicas for use as carrier materials e.g. for choline chloride. One key feature of such silicas for use as carrier materials is their adsorption capacity, which is often related to high BET surface area and porosity. Many development efforts have therefore been directed to maximizing this adsorption capacity of precipitated silica carriers.

Another general problem with silica carriers waiting for a solution occurs during the handling of adsorbate/carrier systems and is associated with the use of volatile organic compounds. Such compounds may easily be lost via evaporation prior to their intended use, e.g. during storage, transport and other handling steps. This decreases the content of the useful organic component in the system and may lead to environmental pollution by VOC emissions, unpleasant smell, and many other negative consequences.

WO2018149756A1 discloses powdery formulations comprising nitrate esters such as 3-nitrooxypropanol (3-NOP) adsorbed on a silica carrier with a particle size D(v, 0.5) of $\leq 320\ \mu m$, e.g. a precipitated silica. It is emphasized in this patent application, that evaporation of 3-NOP during the storage is a challenge. According to the patent application, variation of particle size of the silica carrier may to some extent solve this problem. However, the examples of WO2018149756A1 show that 3-NOP retention was still incomplete, even with the optimized carriers. Hence, the problem of retention of volatile adsorbates on a silica carrier still existed in this case.

Surprisingly, it has now been found that precipitated silica carriers with specific reduced silanol group density may be efficiently used for solving the afore-mentioned problem, i.e. reducing the desorption of volatile compounds from precipitated silica, if used as carriers. The required reduced silanol group density may be achieved e.g. via thermal treatment under particular reaction conditions. It turned out that there is an optimum of absorption capacity of a precipitated silica carrier corresponding to a specific narrow range of silanol group density.

Mentioned above EP1241136A2 shows preparation of several precipitated silicas including their granulation by spray drying of aqueous suspensions. According to the experimental data, silanol group density measured with Sears method, i.e. alkaline titration, does not actually correlate with water content of silica samples. Thus, the samples with the same water content (examples 4 and 9, 6 wt% water) show very different silanol group densities (29.5 vs. 18.4), whereas the samples with similar Sears values (examples 1 and 2, Sears 31.3 vs. 30.9) show very different water contents (6.2 vs. 5.3 wt%). The data (example 9) also suggests that silicas with the lowest silanol group density would lead to the lowest adsorption capacity as confirmed by the DBP adsorption values. This drives the skilled person to the conclusion not to reduce the silanol group density if a better adsorption is desired.

US5,395,605 discloses preparation of precipitated silicas by heat treatment at the temperature of at least 700 °C for at least one minute. Such silicas have "lower water uptake" and are proposed for use as reinforcing fillers with a good dispersibility, for e.g. silicone formulations. US5,395,605 does not suggest using thermally treated silicas as adsorbents.

US20150209252A1 discloses toothpaste compositions, i.e. not free-flowing "dry mixtures", but viscose, pasty liquid compositions, containing typically about 20 wt% of heat-treated in the temperature range 150°C-1000 °C precipitated silicas. These heat-treated silicas show reduced water content and "lower affinity for water" compared to non-heat-treated silicas and are used primarily as abrasive materials.

Similarly, WO2022101015A1 describes precipitated silicas with a number of silanol groups (determined by mass loss in the temperature rage 200°C-800°C) not higher than 2.00 $OH/nm^2$ and CTAB of 5-45 $m^2/g$ prepared by thermal treatment of corresponding "starting silicas" with CTAB of less than 50 $m^2/g$ at 400-650 °C for 1 to 180 min. Such thermally treated silicas are reported to allow less decomposition of hydrogen peroxide in the corresponding toothpaste formulations.

US20160159654A1 discloses producing calcined in the range 600 °C - 1200 °C precipitated silicas having "low hygroscopicity" and high dispersibility in resin.

Technical problem solved by the present invention is that of providing an adsorbate/carrier system based on precipitated silica carrier and containing volatile organic compounds as an adsorbate, wherein the volatile compound shows strong adsorption and reduced desorption / evaporation rate from the carrier. Additionally, the carrier should be characterized by overall high adsorption capacity and be able to adsorb relatively large quantities of the adsorbate and

form free-flowing powdery or granular adsorbate/carrier mixtures. Additionally, low attrition and fines formation is desired as well as low extent of aggregation of the carrier, which would improve handling with such systems.

**Solution**

The present invention provides dry mixture comprising

- from 10 % to 99 % by weight of a precipitated silica with a BET surface area of from 30 $m^2/g$ to 700 $m^2/g$ and having a silanol group density $d_{SiOH}$ determined by reaction of the pre-dried silica with lithium aluminum hydride of from 1.55 to 3.65 $SiOH/nm^2$ and
- 1 wt% to 90 wt% of at least one organic compound, comprising at least one oxygen (O) and/or nitrogen (N) atom and having a vapour pressure of at least 0.01 kPa at 293.15 K, adsorbed on the precipitated silica.

As discussed above, based on the known prior art, the person skilled in the art trying to optimize the adsorption/desorption of polar volatile compounds on precipitated silica carriers would arrive at the conclusion that precipitated silicas with reduced silanol group density, e.g. those prepared by thermal treatment, show lower affinity for and adsorption of polar volatile compounds such as water. This teaches the skilled person away from the idea of using adsorbate/carrier systems with reduced silanol group density of silica carrier.

**Precipitated silica**

The term "dry mixture" means in the context of the present invention powdery or granular free-flowing mixture. Such "dry mixture" typically contains a powdery or granular porous carrier material and an adsorbate adsorbed on the carrier. This typically liquid adsorbate is essentially completely and relatively firmly attached to the surface of the carrier's pores so that essentially no non-adsorbed liquid adsorbate can be found in the space between carrier particles. Thus, "dry mixture" of the present invention differs from any liquid or pasty composition comprising a carrier and a liquid adsorbate in that they are free-flowing and typically contain relatively high content of porous carrier.

The dry mixture according to the invention comprises from 10 % to 99 %, preferably 20 % to 98 %, more preferably 25% to 97%, more preferably 30% to 96%, more preferably 40% to 95%, more preferably 50% to 95% by weight of precipitated silica.

The term "precipitated silica" referred to in the present invention is well known in the art. Precipitated silicas are typically produced by the reaction of an alkaline silicate solution, e.g., sodium silicate, with a mineral acid. Commercially, sulfuric acid is mainly used, although other acids such as hydrochloric acid or carbonic acid (carbon dioxide) can be applied as well. The acid and the sodium silicate solution are usually added simultaneously with agitation to water. Precipitated silica arises when silica is precipitated from this dispersion by the neutralization reaction and the generation of by-product sodium salt (sodium sulfate). The precipitated silica consists of aggregates (secondary particles) of primary (or ultimate) colloidal silica particles. The primary particles are mostly spherical and usually have a diameter in the range between 5 nm and 50 nm. The primary particles in the aggregates are covalently bonded to one another by the formation of siloxane bonds. The aggregates are three-dimensional clusters of these primary particles. The aggregates usually have diameters of up to 500 nm. The aggregates are typically linked into a massive gel network during the preparation process. The aggregates themselves can be physically linked to larger agglomerates of up to 100 μm in diameter by the formation of hydrogen bonds between the silanol groups on their surfaces before milling). The median agglomerate size is typically about 20μm-50 μm in diameter (before milling). Precipitated silicas may be treated in many known ways, e.g. milled, subjected to hydrophobization etc.

The precipitated silica in the invention can have a BET surface area of 30 $m^2/g$ to 700 $m^2/g$, preferably of 50 $m^2/g$ to 600 $m^2/g$, more preferably of 100 $m^2/g$ to 500 $m^2/g$, more preferably of 150 $m^2/g$ to 450 $m^2/g$, most preferably of 150 $m^2/g$ to 250 $m^2/g$. The specific surface area, also referred to simply as BET surface area, can be determined according to DIN 9277:2014 by nitrogen adsorption in accordance with the Brunauer-Emmett-Teller method. BET surface area of the precipitated silica in the above-specified preferred range, particularly between 150 $m^2/g$ and 450 $m^2/g$, turned out to be optimal in terms of maximal adsorption of the organic compound per mass of the silica at acceptable mechanical strength leading to less mechanical attrition and fines formation and better handling.

The silanol group density $d_{SiOH}$ is the average number of silanol groups relative to BET surface area of the silica powder (in nm), expressed in $SiOH/nm^2$. This parameter $d_{SiOH}$ can be determined by the method described in detail on page 8, line 17 thru page 9, line 12 of EP 0725037 A1 by reaction of the silica powder (or granules) with lithium aluminium hydride. Silica is usually pre-dried under vacuum ($10^{-2}$ mbar) for 30 minutes at 130 °C prior to the measurement. The silanol (SiOH) groups of the silica are reacted with lithium aluminium hydride ($LiAlH_4$), the quantity of gaseous hydrogen formed during this reaction and thus the amount of silanol groups in the sample $n_{SiOH}$ (in mmol SiOH/g) is determined. Using the corresponding BET surface area (in $m^2/g$) of the tested material, the silanol group content in mmol SiOH/g can be

easily converted in number dsion of silanol groups relative to BET surface area:

$$d_{OH}\ [SiOH/nm^2] = (n_{SiOH}\ [mmol\ SiOH/g] \times N_A) / (BET\ [m^2/g] \times 10^{21}),$$

wherein $N_A$ is Avogadro number (~6.022*10$^{23}$).

The precipitated silica in the present invention has a silanol group density $d_{SiOH}$ of from 1.55 SiOH / nm$^2$ to 3.65 SiOH / nm$^2$, preferably from 1.60 SiOH / nm$^2$ to 3.65 SiOH / nm$^2$, more preferably from 1.70 SiOH / nm$^2$ to 3.65 SiOH / nm$^2$, more preferably from 1.80 SiOH / nm$^2$ to 3.65 SiOH / nm$^2$, more preferably from 2.00 SiOH / nm$^2$ to 3.60 SiOH / nm$^2$, more preferably from 2.20 SiOH / nm$^2$ to 3.55 SiOH / nm$^2$, more preferably from 2.40 SiOH / nm$^2$ to 3.50 SiOH / nm$^2$, more preferably from 2.60 SiOH / nm$^2$ to 3.45 SiOH / nm$^2$, more preferably from 2.70 SiOH / nm$^2$ to 3.40 SiOH / nm$^2$. Silanol group densities in the above-mentioned narrow range, particularly 2.00 SiOH / nm$^2$ to 3.60 SiOH / nm$^2$ were found to be particularly suitable for the optimal retention of volatile organic compounds adsorbed on the surface of silica carrier. On the other hand, interestingly, precipitated silicas with silanol group densities in a narrow range from 3.35 SiOH / nm$^2$ to 3.65 SiOH / nm$^2$, particularly 3.40 SiOH / nm$^2$ to 3.60 SiOH / nm$^2$were found to show improved retention of volatile compounds while simultaneously having no or only the smallest extent of agglomeration of silica particles, which is a useful feature while handling such systems.

The silica used in the inventive mixture is preferably characterized by a di-(2-ethylhexyl) adipate (DOA) absorption capacity of at least 150 mL DOA / 100g silica, more preferably at least 150 mL DOA / 100g silica. The measurement of DOA absorption capacity of the silica may be done following ISO19246:2016. Thus, preferred precipitated silicas used in the present invention are characterized by relatively high adsorption values of typical organic adsorbates, which differs them from silicas having low BET surface area and/or porosity and/or pore diameters in sub-optimal for adsorption range, i.e. too small or too large pore diameters.

The precipitated silica in the present invention is preferably hydrophilic, i.e. not subjected to hydrophobization by any kind of hydrophobizing surface treatment. As such, the precipitated silica in the invention usually has a relatively low carbon content of less than 3 % by weight, preferably less than 1% by weight, more preferably less than 0.5% by weight, more preferably less than 0.1% by weight. The carbon content can be determined by elemental analysis according to EN ISO3262-20:2000 (Chapter 8). The analysed sample is weighed into a ceramic crucible, provided with combustion additives, and heated in an induction furnace under an oxygen flow. The carbon present is oxidized to $CO_2$. The amount of $CO_2$ gas is quantified by infrared detectors. The stated carbon content of silica powder according to the invention refers to all carbon-containing components of the silica except for non-combustible compounds such as e.g. silicon carbide.

The precipitated silica used in the present invention may be in the form of powder, i.e. fine particles, or preferably in the form of granules, i.e. larger, aggregated particles.

The precipitated silica in the present invention preferably has a numerical median particle size $d_{50}$ of at least 5 µm, more preferably at least 10 µm, more preferably from 50 µm to 1000 µm, more preferably from 100 µm to 800 µm, more preferably from 200 µm to 600 µm, more preferably from 250 µm to 550 µm, more preferably from 300 µm to 500 µm. A numerical median particle size of the silica can be determined according to ISO 13320:2009 by laser diffraction particle size analysis. The resulting measured particle size distribution is used to define the median $d_{50}$, which reflects the particle size not exceeded by 50% of all particles, as the numerical median particle size. Median particle size of the precipitated silica in the above-specified preferred range, particularly between 200 µm and 600 µm, turned out to be optimal in terms of handling of the resulting adsorbate/carrier system, i.e. minimal attrition and fines formation, loading and other aspects.

Tamped densities (also referred to as "tapped density") of various pulverulent or coarse-grain granular materials can be determined according to DIN ISO 787-11:1995 "General methods of test for pigments and extenders -- Part 11: Determination of tamped volume and apparent density after tamping". This involves measuring the apparent density of a bed after agitation and tamping. The precipitated silica in the invention preferably has a tamped density of more than 30 g/L, more preferably of 30 g/L to 600 g/L, more preferably of 50 g/L to 500 g/L, more preferably of 70 g/L to 450 g/L, more preferably of 150 g/L to 450 g/L, more preferably of 200 g/L to 400 g/L. Tamped density of the precipitated silica in the above-specified range, particularly between 150 g/L and 450 g/L, turned out to be optimal in terms of handling of the resulting adsorbate/carrier system, i.e. minimal attrition and fines formation, loading and other aspects.

Loss on drying (LOD) of the precipitated silica in the present invention is preferably less than 5.0 wt%, more preferably less than 4.5 wt%, more preferably less than 4.0 wt%, more preferably less than 3.5 wt%, more preferably less than 3.0 wt%, more preferably less than 2.5 wt%. Loss on drying can be determined after 2 hours at 105°C following ISO 787-2.

LOD value only reflects the partial water content of the silica, as it shows the water being dried off at 105 °C. The total water content of the precipitated silica may be measured by the Karl-Fischer titration method using any suitable Karl-Fischer titrator according to STN ISO 760. Water content of the precipitated silica in the invention determined by Karl-Fischer method is preferably lower than 6.0 % by weight, more preferably from 0.5 % by weight to 5.5 % by weight, more preferably from 0.7 % by weight to 5.2 % by weight, more preferably from 0.9 % by weight to 5.1 % by weight, more

preferably from 1.0 % by weight to 5.0 % by weight, more preferably from 1.5 % by weight to 4.8 % by weight, more preferably from 2.0 % by weight to 4.5 % by weight.

Water content of precipitated silicas may depend on their BET surface area, typically being higher for higher surface areas. Therefore, the ratio of water content to BET surface area, which is independent on BET surface area, sems to be more suitable for characterization of the thermal treated precipitated silicas in the present invention. The ratio of water content of the silica in % by weight, as determined by Karl-Fisher titration to BET surface area of the silica in $m^2/g$ is preferably from 0.005 wt%×g/$m^2$ to 0.030 wt%×g/$m^2$, more preferably from 0.006 wt%×g/$m^2$ to 0.028 wt%×g/$m^2$, more preferably from 0.008 wt%×g/$m^2$ to 0.025 wt%×g/$m^2$, more preferably from 0.010 wt%×g/$m^2$ to 0.023 wt%×g/$m^2$. This above-specified value of water content - to BET ratio is characteristic for precipitated silicas in the inventive mixture having relatively low water content at simultaneously relatively high BET surface area. This set of features is preferred for having both high adsorption and retention of the adsorbed organic compound.

The porosity of precipitated silica particles typically depends on the size of the silica primary particles and the kind of their aggregation and agglomeration. The pores are formed by the spaces between the primary particles, and the aggregates.

The precipitated silica in the inventive mixture is preferably characterized by a total volume of mesopores, i.e. the pores in the diameter range of 2 nm to 50 nm, of 0.65 mUg to 0.90 mUg, as determined by the mercury intrusion method according to DIN ISO 15901-1. The principle of this method firstly described by H.L Ritter and L.C Drake in Ind. Eng. Chem. Anal. Ed. 17 (1945) pp. 782-786 and pp 787-791, is based on measurement of the volume of mercury pressed into a porous solid body as a function of the pressure applied.

The average pore diameter of the pores of the precipitated silica is typically in the range from 2 nm to 40 nm, preferably 3 nm to 30 nm, more preferably 4 nm to 20 nm, as determined by nitrogen adsorption method.

Precipitated silica in the invention preferably has a pH of 2.5 to 7.0, more preferably 3.5 to 6.5, more preferably 3.8 to 6.2, more preferably 4.0 to 6.0, more preferably 4.5 to 5.5. pH value of the silica can be measured in a 5 wt% silica dispersion in water according to ISO 787-9. It turned out that the maximal adsorption and retention of the organic compound is observed in the above-mentioned preferred range of pH, particularly at pH from 4.0 to 6.0.

**Organic compound**

The dry mixture of the invention comprises 1% to 90%, preferably 2% to 50%, more preferably 3% to 40%, more preferably 4% to 30%, more preferably 5% to 20% by weight of at least one organic compound adsorbed on the precipitated silica.

The term "adsorbed" is used in the present invention in its typical, well-known for the person skilled in the art meaning referred to the result of adsorption, i.e. the process in which atoms, ions or molecules of a substance (in the present invention: organic compound) adhere to a surface of the adsorbent.

The term "organic compound" in the context of the present invention is used in its typical, well-known for the person skilled in the art meaning and refers to compounds in which one or more atoms of carbon are covalently linked to atoms of other elements, most commonly hydrogen, oxygen, or nitrogen.

The present invention relates especially to the so-called "volatile" organic compounds adsorbed on the silica carrier. The term "volatile organic compound (VOC)" is used in the context of the present invention in its common, well-known for the skilled person meaning. The VOC-directive [Official Journal of the European Communities; Brussels: 1999. EU VOC-directive: Council Directive 1999/13/EC of 11 March 1999 on the limitation of emissions of volatile organic compounds due to the use of organic solvents in certain activities and installations.] defines a volatile organic compound (VOC) as an organic compound having at 293.15 K (= 20 °C) a vapour pressure of 0.01 kPa (= 0.075 mmHg) or more.

The organic compound in the present invention can have a boiling point of up to 300°C, preferably 20°C-280°C, more preferably 30°C-260 °C, more preferably 50°C-250 °C, more preferably 100°C-220°C, at a pressure of 1 atm. The organic compound is preferably in a liquid form. All pressures referred to in the present invention are absolute pressures.

Volatile organic compounds usually have higher volatility. However, sometimes the compounds having relatively high boiling points may have quite high vapor pressure at 293.15 K (20 °C) and are therefore volatile.

The present invention relates to relatively volatile compounds, whose retention in adsorbed form on the precipitated silica of the dry mixture according to the invention is improved by the invention. Thus, camphor having a boiling point of 209 °C, is reported to have vapor pressure of about 24 Pa at 293.15 K and is therefore volatile according to the definition given above, while naphthalene having a boiling point of 218 °C and a vapor pressure of about 7 Pa at 293.15 Pa is not volatile.

The organic compound comprises at least one oxygen (O) and/or nitrogen (N) atom in its structure. The presence of at least one of these atoms in the organic compound confer certain polarity to such organic compounds and facilitates adsorption of these compounds on precipitated silica by interaction of the corresponding functional groups with silica.

The organic compound preferably has 2 to 12, more preferably 2 to 10, more preferably 3 to 8 carbon atoms in its structure.

The organic compound is preferably selected from the group consisting of unsaturated or saturated linear, branched, cyclic and/or aromatic alcohols, such as propanol, butanol, pentanol, hexanol, cyclohexanol, heptanol, octanol, estragole, ethyl maltol, terpineol, eucalyptol, linalool, dihydromyrcenol; aldehydes such as benzaldehyde; ketones such as camphor; carboxylic acids such as propionic or butyric acid, carboxylic esters such as benzyl acetate, methyl butyrate, ethyl butyrate, hexyl acetate, ethyl acetate, isoamyl acetate, methyl salicylate; ethers such as diethyl ether, nitrate esters such as 3-nitrooxypropanol, primary, secondary or tertiary amines such as propylamine, oxygen and/or nitrogen heterocycles such as furan, pyrrole etc, and the mixtures thereof.

In a preferred embodiment of the invention, the dry mixture comprises a compound of formula (I) as an organic compound

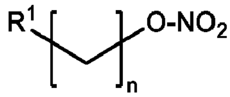

formula (I),

wherein

n is an integer from 1 to 10, more preferably from 2 to 8,
$R^1$ is selected from the group consisting of H, $C_1$-$C_6$ alkyl, phenyl, -OH, $-NH_2$, -CN, - COOH, -O(C=O)$R^2$, and $-ONO_2$, and
$R^2$ is $C_1$-$C_6$ alkyl, phenyl, pyridyl such as preferably 2-pyridyl
with the proviso that when n is > 3 the hydrocarbon chain may be interrupted by -O- or -NH-.

The organic compound is preferably an alcohol, and/or nitrate ester with 2 to 10 carbon atoms.

**Process for producing the dry mixture**

The invention further provides a process for manufacturing the inventive dry mixture, comprising:

- step (a) of thermal treatment of a precipitated silica having a BET surface area of from 30 $m^2$/g to 700 $m^2$/g and a silanol group density $d_{SiOH}$ determined by reaction of the pre-dried silica with lithium aluminum hydride of more than 3.65 SiOH/$nm^2$ at a temperature of from 120°C to 700 °C, for at least 1 minute and

- step (b) of adding at least one organic compound, comprising at least one oxygen (O) atom and/or at least one nitrogen (N) atom, and having a vapour pressure of at least 0.01 kPa at 293.15 K to the thermally treated silica prepared in step (a) and adsorbing the organic compound on the silica.

The process according to the present invention combines thermal treatment of selected precipitated silica under specified conditions (step (a)) and adsorption of selected volatile organic compounds on the pre-treated silica carrier (step (b)).

The precipitated silica used in step (a) of the inventive process is referred to as "starting precipitated silica" in the context of the present invention.

The starting precipitated silica in the invention can have a BET surface area of 30 $m^2$/g to 700 $m^2$/g, preferably of 50 $m^2$/g to 600 $m^2$/g, more preferably of 100 $m^2$/g to 500 $m^2$/g, more preferably of 150 $m^2$/g to 450 $m^2$/g, most preferably of 150 $m^2$/g to 250 $m^2$/g, as determined according to DIN 9277:2014 by nitrogen adsorption in accordance with the Brunauer-Emmett-Teller method.

The starting precipitated silica in the present invention has a silanol group density $d_{SiOH}$ of more than 3.65 SiOH / $nm^2$, preferably from 3.65 SiOH / $nm^2$ to 8.00 SiOH / $nm^2$, more preferably from 3.70 SiOH / $nm^2$ to 6.00 SiOH / $nm^2$, as determined by the method described in detail on page 8, line 17 thru page 9, line 12 of EP 0725037 A1 by reaction of the silica powder (or granules) with lithium aluminium hydride.

The starting precipitated silica in the present invention preferably has a numerical median particle size $d_{50}$ of at least 5 $\mu$m, more preferably at least 10 $\mu$m, more preferably from 50 $\mu$m to 1000 $\mu$m, more preferably from 100 $\mu$m to 800 $\mu$m, more preferably from 200 $\mu$m to 600 $\mu$m, more preferably from 250 $\mu$m to 550 $\mu$m, more preferably from 300 $\mu$m to 500 $\mu$m, as determined according to DIN ISO 787-11:1995.

It is believed that thermal treatment in step a) of the inventive process leads to reducing the silanol group density by condensation of such groups and formation of O-Si-O bridges.

It has been found that the extent of the required silanol group density reduction of the precipitated silica in step (a) of the inventive process depends on the combination of (1) the temperature of thermal treatment and (2) duration of thermal

treatment.

Step (a) is carried out at 120 °C to 700 °C, preferably at 120°C to 600 °C, more preferably at 120 °C to 500 °C, more preferably at 120 °C to 450 °C, more preferably at 150 °C to 450 °C, more preferably at 200 °C to 400 °C.

The duration of step (a) is at least 1 minute, preferably at least 5 minutes, more preferably at least 10 minutes, more preferably at least 30 minutes, more preferably at least 1 hour.

Step (a) of the inventive process is preferably carried out at a temperature of from 120 °C to 450 °C for at least 10 minutes.

Importantly, the same extent of the silanol group density reduction may be achieved using the higher temperature and the shorter duration of the thermal treatment or using the lower temperature and the longer duration of the process. Thus, process conditions may be adjusted within the specified temperature and duration ranges so that the required silanol group density of the precipitated silica can be achieved.

Thermal treatment in step (a) of the inventive process can be carried out under a gas atmosphere of typically at least 10 mbar, preferably at least 100 mbar, more preferably at least 500 mbar, more preferably 0.5 bar to 2.0 bar, more preferably 0.8 bar to 1.2 bar. Thus, both reduced pressure (< 1 bar) and overpressure compared to atmospheric pressure may be used in the inventive process.

Any suitable gas, preferably the one not reacting with precipitated silica under process conditions, may constitute such gas atmosphere. Dried air and/or nitrogen are preferably used as gases in the process.

Step (a) of the inventive process may be carried out batchwise under the above-mentioned suitable gases or continuously, in the latter case preferably under continuous flow of such suitable gases. The preferred ratio of the employed total gas volume (under standard conditions) to the employed total mass of the precipitated silica is in the range 0.1 $m^3$/kg to 10 $m^3$/kg, more preferably 0.3 $m^3$/kg to 6 $m^3$/kg, more preferably 0.5 $m^3$/kg to 3 $m^3$/kg. In a continuous process, the above-mentioned ratio of the employed total gas volume (under standard conditions) to the employed total mass of the precipitated silica corresponds to the ratio of gas flow (in $Nm^3$/h) to silica mass flow (kg/h).

Essentially no water except for the adsorbed water contained in the (starting) precipitated silica prior to carrying out step (a) of the process, is preferably added before, during or after carrying out step a) of the inventive process. Small amounts of water may be introduced into the process during thermal treatment in step (a) e.g. as humidity with the used carrier gas. However, larger amounts of water, e.g. treatment silica with steam or saturated with water air, are not recommended. "Essentially no water" means in the context of the present invention that the total amount of water introduced before, during or after carrying out step (a) does not exceed 50%, preferably does not exceed 20%, more preferably does not exceed 10% of the total amount of water removed from the starting precipitated silica during the thermal treatment process.

In this way, the undesired adsorption of water on the precipitated silica can be avoided in the process and the required reduction of the silanol group density may be achieved at relatively low process temperatures, which in turn allows keeping the BET surface area and the adsorption capacity of the silica on the high level.

Gas atmosphere during the thermal treatment process step (a) preferably has maximal water content of 0.5 g water/L gas, preferably maximally 0.3 g water/L gas, more preferably maximally 0.1 g water/L gas, more preferably maximally 0.05 g water/L gas.

The thermal treatment in step (a) is preferably carried out under movement of the silica, at least temporally during the thermal treatment step a), at the motion rate of at least 1 cm/min, more preferably at least 5 cm/min, more preferably at least 25 cm/min, more preferably at least 50 cm/min. It has been found that such a movement of silica during the thermal treatment process leads to much less aggregation, better temperature control and more homogeneous product properties.

The inventive process can be carried out in any suitable apparatus allowing keeping the precipitated silica particles at the above-specified temperature for a specified period of time, while moving the silica. Some suitable apparatuses are fluidized bed reactors and rotary kilns. Rotary kilns, particularly those with a diameter of 1 cm to 2 m, preferably 5 cm to 1 m, more preferably 10 cm to 50 cm, are preferably used in the inventive process. The silica can be moved at least temporally during the thermal treatment step a), but preferably, the silica is being moved at the specified motion rate continuously for the whole duration of the thermal treatment step (a) of the inventive process. The motion rate in a rotary kiln corresponds to circumferential speed of this reactor type. The motion rate in a fluidized bed reactor corresponds to the carrier gas flow rate (fluidization velocity).

Precipitated silica obtained in step (a) of the process can be cooled down to 15 °C -200 °C, more preferably 20°C - 50 °C, prior to carrying out step (b) of the process. This cooling step is preferably carried out under exclusion of water, e.g. in a flow of dried gas such as air or nitrogen.

In step b) of the inventive process, the precipitated silica subjected to thermal treatment in step a) and optionally cooled down to 15 °C -200 °C, preferably 20°C - 50 °C, is subjected to adding at least one organic compound, comprising at least one oxygen (O) atom and/or at least one nitrogen (N) atom, and having a vapour pressure of at least 0.01 kPa at 293.15 K and adsorbing of the organic compound on the silica.

Step (b) of the process preferably directly follows step (a) and optional cooling the silica, i.e. no longer transport

and/or storage of silica between steps (a) and (b) of the process takes place. In any case, silica thermally treated in step (a) of the process is preferably kept under essential exclusion of water until adsorption of the organic compound in step (b) takes place. In this case, the reduced silanol group density of the thermally-treated precipitated silica can be efficiently kept until the adsorption step.

Step b) of the process according to the invention can be carried out at a temperature of 10 °C to 250 °C, preferably 20°C to 50 °C for 1 second to 24 hours, preferably 1 minute to 1 hour.

The organic compound may be added in any suitable way, e.g. by spraying ensuring the homogeneous distribution thereof on the silica surface.

Step (b) can be conducted under exclusion of water and/or under protective gas, such as, for example, nitrogen. This step can be carried out in any suitable apparatus such as mixer equipped with a spraying means, either continuously or batchwise.

**Use of the mixture**

The inventive mixture can be used as food and/or animal feed additive, for transport and/or storage of chemicals and chemical intermediates, as detergent, additive for washing machines, as lack additive, as component of rubber or sealant, e.g. silicone compositions, as perfuming or flavoring ingredient, and in toothpaste.

Preferably, the mixture according to the invention can be used as such or as a component of animal feed products or in the production of feed products.

Most preferably, the inventive mixture comprising the compound of formula (I) described above is used as a component of animal feed products, especially for reducing methane production by ruminants (e.g. cattle, sheep, goats).

Especially in the case if the mixture according to the present invention is used a component of a animal feed product, it can additionally be coated with customary in the art coatings such as wax or fats. If present, such coating is generally applied in amounts of 2 to 50 wt.-% based on the total weight of the mixture. Advantageously, the coating comprises at least one wax and/or at least one fat, which has a dropping point of from 30 to 85 °C. The dropping point of a material as used herein refers to the temperature (in °C) when the material begins to melt under standardized conditions. Thus, the material is heated so long until it changes the state of matter from solid to liquid. The dropping point is the temperature when the first dropping is released from the material. The determination of the dropping point ("Tropfpunkt") is carried out as described in the standard norm DIN ISO 2176.

Particularly suitable waxes to be used as coating in the context of the present invention include organic compounds consisting of long alkyl chains, natural waxes (plant, animal) which are typically esters of fatty acids and long chain alcohols as well as synthetic waxes, which are long-chain hydrocarbons lacking functional groups.

Particularly suitable fats to be used as coating in the context of the present invention include a wide group of compounds which are soluble in organic solvents and largely insoluble in water such as hydrogenated fats (or saturated fats) which are generally triesters of glycerol and fatty acids. Suitable fats can have natural or synthetic origin. It is possible to hydrogen- ate a (poly)unsaturated fat to obtain a hydrogenated (saturated) fat.

Preferred examples of waxes and fats to be used as coating according to the present invention are glycerine monostearate, carnauba wax, candelilla wax, sugarcane wax, palmitic acid, stearic acid hydrogenated cottonseed oil, hydrogenated palm oil and hydrogenated rapeseed oil as well as mixtures thereof.

Another preferred use of the inventive dry mixture is that as a perfuming or flavouring ingredient, e.g. in the corresponding compositions. A perfuming or flavouring ingredient, within the framework of the invention, designates a compound typically used in perfumery or flavour industry able to impart an odour, or to improve, enhance or modify the odour or taste properties of the composition to which it is added. Usually, these ingredients will form a complex mixture of volatile ingredients of natural or synthetic origin. The nature of these ingredients can be found in specialised books, e.g. in S. Arctander (Perfume and Flavor Chemicals, Montclair N.J., USA 1969), or in Fenaroli's Handbook of Flavour Ingredients, CRC Press or yet in Synthetic Food Adjuncts by M.B. Jacobs, van Nostrand Co., Inc. or similar textbooks of reference. Typical perfuming or flavouring ingredients are typically used in liquid form.

**Examples**

**Analytical methods**

The number of silanol groups relative to BET surface area $d_{SiOH}$ [in SiOH/nm$^2$] was determined by reaction of the pre-dried samples of silica with lithium aluminium hydride solution as described in detail on page 8, line 17 thru page 9, line 12 of EP 0725037 A1.

Specific BET surface area [in m$^2$/g] was determined according to DIN 9277:2014 by nitrogen adsorption in accordance with the Brunauer-Emmett-Teller method.

Water content [in wt.%] was determined by Karl Fischer titration using a Karl Fischer titrator.

Tamped density [in g/L] was determined according to DIN ISO 787-11:1995 "General methods of test for pigments and extenders -- Part 11: Determination of tamped volume and apparent density after tamping".

Oil absorption of precipitated silica (DOA absorption, in mU 100g) was determined using di-(2-ethylhexyl) adipate (DOA) according to ISO 19246:2016.

Pore volume [mUg] for mesopores (2-50 nm) was determined by nitrogen adsorption method according to DIN 66134.

**Silica samples**

Silica used in Comparative Example 1 was precipitated silica Sipernat® 2200 (manufacturer: Evonik Operations GmbH).

**Preparation of silica samples for Examples 2-6 and Comparative example 7**

Precipitated silica Sipernat® 2200 (manufacturer: Evonik Operations GmbH) was subjected to thermal treatment in a rotary kiln of ca. 160 mm diameter and 2 m length at a temperature from 120 °C to 900 °C (specified in the Table 1). The mean residence time of the silica in the rotary kiln was 1 hour. Rotational speed was set to 5 rpm resulting in a throughput of approximately 1 kg/h of silica. Dry and filtered compressed air was fed continuously with a flow rate of ca. 1 $m^3/h$ to the kiln outlet (in counterflow to the thermally treated silica flow) to provide preconditioned air for the convection in the tube. The process was smooth. No clogging of the rotary kiln was observed. Physico-chemical properties of the obtained thermally treated silica are shown in Table 1.

**Absorption of 1-propanol on silica and determination of the extent of the desorption (1-propanol retention) after 24 h [%]**

1-Propanol (10.0 % by weight of tested silica, ca. 1 g) was sprayed at ambient temperature on ca. 10 g of the silica samples directly after preparation and cooling under flow of dried air. After thorough mixing the silica, a sample thereof (M ~ 1 g) was taken, weighted ($m_0$), and spread in a thin layer in a petri dish. After 24 hours staying under air (relative humidity ca. 50%) at 25 °C in fume hood, the sample was weighted again ($m_1$). Mass difference ($m_1$-$m_0$) was attributed to desorbed/evaporated 1-propanol. The value "1-propanol retention after 24 h [%]" shown in the table below was calculated as $100\%\times[1-(m_0-m_1)\times/(0.1\times M)]$.

**Results**

Surprisingly, it turned out that the extent (rate) of 1-propanol desorption from silica depended on the silanol group density of silica used for alcohol absorption. The results shown in Table 1 indicate the optimum of 1-propanol retention at silanol group density of the precipitated silica of about 1.75 to 3.60 SiOH/$nm^2$ (examples 2 to 6), particularly 2.75 to 3.34 SiOH/$nm^2$ (examples 3 and 4). Interestingly, relatively high silanol group density of the untreated precipitated silica (3.73, Comparative Example 1) delivered inferior results of 1-propanol retention just as the relatively low silanol group density of the silica calcined at 900 °C (1.40, Comparatve Exaple 7). The lowest aggregation rate of silica was observed in the range of silanol group density above 3.34 and below 3.73 SiOH/$nm^2$.

**Table 1**

| Example | Thermal treatment temperature [°C] | BET [$m^2$/g] | KF Water content [wt%] | Silanol density [SiOH / $nm^2$] | $d_{50}$ [μ-m] | Tamped density [g/L] | Pore volume (2-50 nm) [mL/g] | DOA Absorption [mL/100 g] | 1-Propanol retention after 24 h [%] |
|---|---|---|---|---|---|---|---|---|---|
| Comparative Example 1 | No | 180 | 6.92 | 3.73 | 320 | 258 | 0,79 | 225 | 20 |
| Example 2 | 120 | 182 | 5.10 | 3.60 | 330 | 254 | 0,77 | 221 | 56 |
| Example 3 | 300 | 187 | 4.20 | 3.34 | 480 | 242 | 0,76 | 210 | 67 |
| Example 4 | 400 | 189 | 2.70 | 2.75 | 479 | 240 | 0,84 | 207 | 68 |
| Example 5 | 500 | 191 | 2.49 | 2.07 | 484 | 259 | 0,77 | 202 | 53 |
| Example 6 | 700 | 172 | 0.90 | 1.75 | 512 | 261 | 0,78 | | 44 |

(continued)

| Example | Thermal treatment temperature [°C] | BET [$m^2$/g] | KF Water content [wt%] | Silanol density [SiOH / $nm^2$] | $d_{50}$ [μ-m] | Tamped density [g/L] | Pore volume (2-50 nm) [mL/g] | DOA Absorption [mL/100 g] | 1-Propanol retention after 24 h [%] |
|---|---|---|---|---|---|---|---|---|---|
| Comparative Example 7 | 900 | 110 | 0.46 | 1.40 | 953 | | 0,63 | | 15 |

## Claims

1. Dry mixture comprising

   - from 10 % to 99 % by weight of a precipitated silica with a BET surface area of from 30 $m^2$/g to 700 $m^2$/g and having a silanol group density $d_{SiOH}$ determined by reaction of the pre-dried silica with lithium aluminum hydride of from 1.55 to 3.65 SiOH/$nm^2$and
   - 1 wt% to 90 wt% of at least one organic compound, comprising at least one oxygen (O) and/or nitrogen (N) atom and having a vapour pressure of at least 0.01 kPa at 293.15 K, adsorbed on the precipitated silica.

2. The mixture according to claim 1, wherein the ratio of water content of the silica in % by weight, as determined by Karl-Fisher titration to BET surface area of the silica in $m^2$/g is from 0.005 wt%*g/$m^2$ to 0.030 wt%*g/$m^2$.

3. The mixture according to claims 1-2, wherein the precipitated silica has a numerical median particle size $d_{50}$ determined by laser diffraction method of from 200 μm to 600 μm.

4. The mixture according to claims 1-3, wherein the silica has a di-(2-ethylhexyl) adipate (DOA) adsorption of at least 150 mL DOA/ 100g silica.

5. The mixture according to claims 1-4, wherein the silica is **characterized by** silanol group density $d_{SiOH}$ determined by reaction of the pre-fried silica with lithium aluminum hydride of from 3.35 SiOH/$nm^2$ to 3.65 SiOH/$nm^2$.

6. The mixture according to claims 1-5, wherein the silica has tamped density of 150 g/L to 450 g/L.

7. The mixture according to claims 1-6, wherein the silica has a pH of the 5 wt% aqueous dispersion of the silica in the range 4.0 to 6.0.

8. The mixture according to claims 1-7, wherein the organic compound is selected from the group consisting of unsaturated or saturated, linear, branched, cyclic and/or aromatic alcohols, aldehydes, ketones, carboxylic acids, carboxylic esters, ethers, nitrate esters, primary, secondary or tertiary amines, oxygen and/or nitrogen heterocycles, and the mixtures thereof.

9. The mixture according to claims 1-8, wherein the organic compound is an alcohol, diol and/or nitrate ester with 2 to 12 carbon atoms.

10. A process for manufacturing the dry mixture according to claims 1-9, comprising:

    - step (a) of thermal treatment of a precipitated silica having a BET surface area of from 30 $m^2$/g to 700 $m^2$/g and a silanol group density $d_{SiOH}$ determined by reaction of the pre-dried silica with lithium aluminum hydride of more than 3.65 SiOH/$nm^2$ at a temperature of from 120°C to 700 °C, for at least 1 minute and
    - step (b) of adding at least one organic compound, comprising at least one oxygen (O) atom and/or at least one nitrogen (N) atom, and having a vapour pressure of at least 0.01 kPa at 293.15 K to the thermally treated silica prepared in step (a) and adsorbing the organic compound on the silica.

11. The process according to claim 10, wherein the temperature during the thermal treatment is from 120 °C to 450 °C and the duration of the thermal treatment in step (a) is at least 10 minutes.

**12.** The process according to claims 10-11, wherein essentially no water except for the adsorbed water contained in the precipitated silica prior to carrying out step (a) of the process, is added before, during or after carrying out step a) of the process.

**13.** The process according to claims 10-12, wherein step (a) is carried out under an atmosphere of gas such as air or nitrogen with a maximal water content of 0.5 g water/L gas.

**14.** The process according to claims 10-13, wherein the thermal treatment in step (a) is carried out under movement of the silica at the motion rate of at least 1 cm/min, preferably in a rotary kiln or a fluidization bed reactor.

**15.** Use of the mixture according to claims 1-9 as food or animal feed additive, for transport and/or storage of chemicals and chemical intermediates, as detergent, additive for washing machines, as lack additive, as component of rubber or sealant compositions, as perfuming or flavoring ingredient, and/or in toothpaste.

Europäisches Patentamt
European Patent Office
Office européen des brevets

# EUROPEAN SEARCH REPORT

Application Number
EP 23 02 0379

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/136672 A1 (PANZ CHRISTIAN [DE] ET AL) 28 May 2009 (2009-05-28) | 1-6,9-14 | INV. C01B33/18 |
| A | * paragraph [0209]; claim 3 * | 15 | |
| | ----- | | |
| X | US 6 191 122 B1 (OELMUELLER ROLF [DE] ET AL) 20 February 2001 (2001-02-20) | 1-14 | |
| A | * column 5, lines tables 1, 2 - column 5, lines 60-65; claims 1, 5 * | 15 | |
| | ----- | | |

TECHNICAL FIELDS SEARCHED (IPC)

C01B

The present search report has been drawn up for all claims

1

| Place of search | Date of completion of the search | Examiner |
|---|---|---|
| The Hague | 31 January 2024 | Mattheis, Chris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 02 0379

This annex lists the patent family members relating to the patent documents cited in the above-mentioned European search report. The members are as contained in the European Patent Office EDP file on
The European Patent Office is in no way liable for these particulars which are merely given for the purpose of information.

31-01-2024

| Patent document cited in search report | Publication date | Patent family member(s) | Publication date |
|---|---|---|---|
| US 2009136672 A1 | 28-05-2009 | AT E537110 T1 | 15-12-2011 |
| | | BR PI0819910 A2 | 13-06-2017 |
| | | CN 101873995 A | 27-10-2010 |
| | | EP 2219996 A1 | 25-08-2010 |
| | | ES 2378120 T3 | 09-04-2012 |
| | | JP 5295261 B2 | 18-09-2013 |
| | | JP 2011510891 A | 07-04-2011 |
| | | KR 20100108534 A | 07-10-2010 |
| | | MY 148331 A | 29-03-2013 |
| | | PL 2219996 T3 | 31-05-2012 |
| | | PT 2219996 E | 05-03-2012 |
| | | TW 200938485 A | 16-09-2009 |
| | | US 2009136672 A1 | 28-05-2009 |
| | | WO 2009068379 A1 | 04-06-2009 |
| US 6191122 B1 | 20-02-2001 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1241136 A2
- WO 2018149756 A1
- US 5395605 A
- US 20150209252 A1
- WO 2022101015 A1
- US 20160159654 A1
- EP 0725037 A1


### Non-patent literature cited in the description


- **H.L RITTER** ; **L.C DRAKE**. *Ind. Eng. Chem. Anal. Ed.*, 1945, vol. 17, 782-786, 787-791
- EU VOC-directive: Council Directive 1999/13/EC. *Official Journal of the European Communities; Brussels*, 11 March 1999